Europäisches Patentamt

European Patent Office

Office européen des brevets

(11) Publication number: **0 217 572 B1**

# EUROPEAN PATENT SPECIFICATION

(45) Date of publication of patent specification: **02.09.92**

(51) Int. Cl.⁵: **A61K 37/36**, A23K 1/165, //A61K37/43

(21) Application number: **86306992.8**

(22) Date of filing: **10.09.86**

(54) Improving carcass quality.

(30) Priority: **10.09.85 AU 2356/85**

(43) Date of publication of application:
**08.04.87 Bulletin 87/15**

(45) Publication of the grant of the patent:
**02.09.92 Bulletin 92/36**

(84) Designated Contracting States:
**AT BE CH DE FR GB IT LI LU NL SE**

(56) References cited:
**EP-A- 0 085 036**
**EP-A- 0 104 933**
**EP-A- 0 192 360**
**EP-A- 0 193 515**
**EP-A- 0 201 219**

**PROC.-ANNU. RECIPROCAL MEAT CONFER-ENCE AM. MEAT SCI. ASSOC. NATL. LIVE STOCK MEAT BOARD, vol. 37, 1984, pages 12-18, US; R.F. OLSEN: "Potential for growth hormones and growth hormone releasing factors to improve carcass composition"**

(73) Proprietor: **FIDELITY FOOD TECHNOLOGIES PTY. LTD.**
**616 St. Kilda Road**
**Melbourne, Victoria(AU)**

(72) Inventor: **Brandon, Malcolm Roy**
**14 Castella Street**
**Ivanhoe East Melbourne, Victoria 3079(AU)**
Inventor: **Walker, Ian James**
**49 Banff Street**
**Corowa New South Wales 2646(AU)**

(74) Representative: **Harding, Richard Patrick et al**
**Arthur R. Davies & Co. 27 Imperial Square**
**Cheltenham GL50 1RO(GB)**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid (Art. 99(1) European patent convention).

JOURNAL OF ANIMAL SCIENCE, vol. 60, no. 1, 1985, pages 118-130; C.S. CHUNG et al.: "Stimulation of swine growth by porcine growth hormone"

JOURNAL OF ANIMAL SCIENCE, vol. 35, no. 4, 1972, pages 794-800; L.J. MACHLIN: "Effect of porcine growth hormone on growth and carcass composition of the pig"

COMMONWEALTH AGRICULTURAL BUREAU, abstract no. 85489825, 0A054-00922, Animal Breeding Abs.; I.D. JOHNSSON et al.: "The effects of exogenous bovine growth hormone and bromocriptine on growth, body development, fleece weight and plasma concentrations of growth hormone, insulin and prolactin in female lambs", & ANIMAL PRODUCTION, vol. 41, no. 2, 1985, pages 207-217

JOURNAL OF ANIMAL SCIENCE, vol. 56, no. 6, 1983, pages 1315-1323; L.A. MUIR et al.: "Effects of exogenous growth hormone and diethylstibestrol on growth and carcass composition of growing lambs"

## EP 0 217 572 B1

### Description

This invention relates to animal husbandry, and in particular to improving the quality of livestock carcasses.

The quality of meat products is critically related to livestock husbandry and feeding practices, and is an important factor in consumer choice. The desirability to the consumer of a particular type of meat depends upon such parameters as price, tenderness, and appearance.

Nutritional factors, including the perceived "healthiness" of the meat, are an increasingly important influence in this area, and among these a high ratio of lean meat to fat is important.

A high ratio of lean meat to fat is also advantageous in the processing of the meat, since extensive trimming of fat and disposal of fat is avoided.

In addition, a high ratio of lean to fat is advantageous for economy of feed conversion, in that typically greater than four times the feed requirement is necessary to deposit 1 kg of fat compared to that necessary to deposit 1 kg of lean tissue.

In many countries the nature of the feed raw material and the genotype of the animal result in a relatively inefficient conversion of feed meat, and in an undesirably high proportion of fat both on the surface and within the muscle.

Attempts to improve the ratio of lean to fat have been made using conventional breeding strategies. These have often been expensive, long-term, and not without undesirable side effects, such as deterioration in meat quality and increase in susceptibility of the target animal to stress.

Specific treatment of animals prior to slaughter, such as intravenous injection of proteolytic enzymes (Australian Patent No. 230,148), or passing electrical current through the body between stunning and exsanguination (Australian Patent No. 529,833), has also been used in an attempt to improve tenderness of meat. However, these methods have generally not been economically viable.

It is an object of the present invention to overcome or at least alleviate, one or more of the difficulties related to the prior art.

According to the present invention there is provided a method for the treatment of an animal to improve carcass quality and food conversion efficiency, which method is characterised in that an exogenous, homologous growth hormone selected from natural or synthetic growth hormone, analogues, derivatives, or fragments thereof is administered to an animal to be treated selected from entire male, female and castrated male pigs, entire male, female and castrated male sheep, and entire male, female and castrated male bovine animals at a preselected liveweight and at a preselected constant dose rate depending on the species and sex of the animal, and in the absence of zearalin;

wherein, when the animal is a pig, the administration is initiated at a liveweight of 35 to 100 kg, and, when the pig is female or a castrated male, the dose rate is 0.06 to 0.10 mg/kg liveweight/day, and, when the pig is an entire male, the dose rate is 0.1 to 0.15 mg/kg liveweight/day;

wherein, when the animal is a bovine animal, administration is initiated at a bodyweight of 200 to 500 kg, and, when the bovine animal is a female or a castrated male, the dose rate is 0.06 to 0.10 mg/kg liveweight/day, and, when the bovine animal is an entire male, the dose rate is 0.10 to 0.15 mg/kg liveweight/day,

and wherein, when the animal is a sheep, administration is initiated at a bodyweight of 15 to 50 kg, and, when the sheep is female or a castrated male, the dose rate is 0.06 to less than 0.10 mg/kg liveweight/day, and, when the sheep is an entire male, the dose rate is 0.10 to 0.15 mg/kg liveweight/day.

Preferably the source of growth hormone is growth hormone in a unit dosage form.

It is to be clearly understood that the use of biologically active fragments, analogues, derivatives or metabolites of growth hormone, and the veterinarily acceptable salts thereof, as well as the use of transgenic animals, are within the scope of the invention.

As stated above, the method according to the present invention may be applied to transgenic animals. For such animals, administration of growth hormone may comprise the controlled use of an initiator (promoter enhancer) fused to the growth hormone gene.

Similarly, although the invention is described particularly with reference to administration of growth hormone, (also known as somatotrophin) it will be apparent that other methods which increase the level of circulating growth hormone, such as administration of growth hormone releasing hormone (GHRH), or precursors of growth hormone such as preprogrowth hormone or progrowth hormone, or, in some animal species, thyrotropin releasing hormone, are within the scope of the invention. Reduction of inhibitory factors against growth hormone, such as somatostatin, or increasing mediators of growth hormone action such as somatomedin, is also contemplated.

Further, where synthetic growth hormone is used, a recombinant growth hormone is preferred. A recombinant growth hormone selected from methionyl AA1-190 and AA4-190 has been found to be suitable for pigs.

Although it is well known that growth hormone (GH) plays an important part in the control of normal growth, its precise role in the control of growth and of metabolism in domestic animals is not well understood. However, it is known that generally the administration of exogenous GH tends to increase the growth rate in all species studied. The role of GH in domestic animals has been briefly reviewed (C.G. Scanes: Monsanto Symposium on Present and Future Trends in Animal Nutrition and Feed Manufacturing Technology, Arkansas, 1983).

Pigs currently provide an increasingly important source of meat in a large number of countries. According to usual practice, weaner pigs weighing 25 to 30 kg are transferred to grower sheds. Growth rate is relatively constant between 30 and 90 kg, but feed consumption increases significantly. Thus the economy of feed conversion progressively deteriorates as the animals approach the slaughter weight of approximately 90 to 110 kg. During the latter growth phase (60 to 110 kg), the carcass develops its final characteristics, and an increasing proportion of the weight gain is represented by fat. Since the energy requirement for production of fat is approximately 2.4 times greater than that for the production of lean tissue, it is clearly advantageous if a metabolic shift toward production of lean tissue can be achieved.

Several studies have investigated the effect of GH on pigs, with considerable variation in results. Turman and Andrews (J. Animal Sci. (1955) 44 7) injected 45 kg pigs daily with porcine GH, and found that treated pigs showed more efficient feed conversion and had leaner carcasses than controls. However, no increase in total weight gain was observed. Moreover, four out of eleven treated pigs died during the experiment as a result of liver and kidney degeneration. Another study (K.D. Lind, R.D. Howard, D.H. Kropf and B.A. Koch: J. Animal Sci. (1968) 27 1763) showed no effect on 72 kg pigs following treatment with porcine GH.

In a more detailed series of experiments, pigs of initial weight approximately 45 kg were treated daily with a range of GH concentration (L.J. Machlin: J. Animal Sci. (1972) 35 794) until they attained a weight of approximately 95 kg. Daily intramuscular injection of 0.22 or 1.10 mg porcine GH per kg bodyweight resulted in high mortality, with liver and kidney degeneration, gastric haemorrhage, oedema and arthritis. Doses of 0.033, 0.066 or 0.132 mg/kg/day resulted in improved average daily weight gain, efficiency of feed conversion, and lean/fat ratio, although the significance of the differences was variable. Furthermore, in 95 kg pigs fed a restricted high protein diet, GH treatment at 0.13 mg/kg/day improved the rate of weight gain compared to untreated controls.

More recently, it has been found that daily intramuscular injection for 30 days of 0.022 mg GH/kg liveweight in male pigs initially weighing 32 kg increased the growth rate by about 10%, and improved feed efficiency. There was no differences between treated and control groups with respect to dressing percentage, marbling score, loin eye area, or backfat thickness, while percentage lipid in the longissiums muscle was increased. There were no adverse effects of treatment on the animals.

In a review article of the state of the art with respect to growth hormone and its use to improve carcass compositions (Proc. Ann. Recip. Meat Conference Am. Meat Sci. Assoc. Natl. Live Stock Meat Board 37, 1984, 12-18) the author acknowledges the apparent attractiveness of growth hormone in the treatment of animals but concludes that GH will never come into general use as a growth promotant unless some significant problems are solved. No relationship of sex, dose rate and stage of initiation is disclosed or even suggested in this article.

Another study (Commonwealth Agricultural Bureau, Abstract No. 85489825, OA054-00922, Animal Breeding Abstracts, I.D. Johnson et al) female lambs were treated with bovine GH at a dose rate of 0.1 mg/kg/day. This study did not consider a homologous treatment, and did not disclose any interrelationship between the initiation of treatment, dose rate and sex of the animal.

A further article (J. Animal Sci. (1983) 56, 6, 1315-1323, L.A. Muir et al) illustrates the level of confusion and conflicting data typical in the art. The study was a comparison between the effects of ovine growth hormone and diethylstilbestrol (DES) on wether lambs in an eight week trial. The results reported were that ovine GH improved feed conversion but did not alter average daily gain or feed intake. Presumably treatment at high levels has no positive effect but merely subjects the animals to the negative effects of high doses of GH.

We have now found that supplementing endogenous growth hormone levels in pigs, sheep and cattle results in improved carcass quality, as assessed by backfat thickness, fat and lean percentage, or loin eye area, optionally together with improved feed conversion efficiency.

No deleterious effect upon the health of the animals was observed, and this was confirmed by pathological examination of all the body tissues of the animals.

In a preferred embodiment wherein the animal is a pig, the administration is initiated at a liveweight of 50 to 100 kg.

Within the preferred initiation range the improvement in carcass quality is essentially similar to that achieved over the longer period. A greater relative improvement in feed conversion efficiency is noted.

Improvement in carcass quality can be measured as an overall increase in lean tissue content together with an increase in the proportion of lean tissue content may be attributed to an increased overall growth rate, an anabolic effect or a combination thereof.

The increase in the proportion of lean tissue to fat tissue may be attributed both to the anabolic effect and a decrease in fat tissue content.

Specifically, we have surprisingly found that there is a previously unsuspected critical interaction between dose of GH, the time of commencing treatment, the length of treatment, dietary energy supply, and sex of pig. The improved carcass quality in female and castrated male pigs, sheep and cattle was particularly unexpected, since normally they lay down fat more readily than entire males and produce an inferior carcass.

Our studies have shown that treatment according to the present invention enhances the growth rate in treated animals relative to controls. Moreover, the carcass quality is greatly improved and the feed consumption of the animals is lower, as a result of the more efficient utilization of feed by animals treated with GH. At supra-optimal doses of GH, growth rate is not further enhanced, as apparently an appetite-suppressing effect can become limiting.

Results are essentially similar in terms of carcass quality if GH is given when the pigs initially weigh 50 to 60 kg to those found when treatment is given over a longer period, for example from 35 kg. Surprisingly, it is not necessary to increase the dose rate of GH in order to attain the same improvement in carcass quality over the shorter period. However, an improvement in feed efficiency is more apparent in treated animals over the 35 to 60 kg range.

We have found a striking increase in the proportion of lean meat in treated pigs. In a carcass of 60 to 65 kg dressed weight, for example, this represents an increase of 4 to 6 kg in the amount of meat, especially in the commercially valuable parts of the carcass. The economic desirability of such an improvement is clear.

In an alternative preferred embodiment for bovine animals, the administration is initiated at a liveweight of 250 to 300 kg. Within the preferred initiation range the improvement in carcass quality is essentially similar to that achieved over the longer period. A greater relative improvement in feed conversion efficiency is noted.

In a further alternative preferred embodiment for sheep, the administration is initiated at a liveweight of 25 to 35 kg. Within the preferred initiation range the improvement in carcass quality is essentially similar to that achieved over the longer period. A greater relative improvement in feed conversion efficiency is noted.

As discussed above, it has surprisingly been found that, within specified parameter ranges for particular animals, an increase in lean tissue content together with an increase in the proportion of lean tissue to fat tissue may be achieved.

The invention also provides a method of decreasing fat content of an animal carcass, which method is characterised in that an exogenous, homologous growth hormone selected from natural or synthetic growth hormone, analogues, derivatives or fragments thereof is administered to an animal to be treated at a generally constant dose rate and in the absence of zearalin;

wherein, when the animal is a female pig, the dose rate is 0.06 to 0.1 mg/kg liveweight/day and the treatment continues for approximately 20 days prior to slaughter, when the animal is an entire male pig, the dose rate is 0.1 to 0.15 mg/kg liveweight/day and the treatment continues for approximately 20 days prior to slaughter, and, when the animal is a castrated male pig, the dose rate is 0.06 to 0.1 mg/kg liveweight/day and the treatment continues for approximately 10 days to at least about 20 days prior to slaughter;

wherein, when the animal is a bovine female, the dose rate is 0.06 to 0.10 mg/kg liveweight/day and the treatment continues for 25 days prior to slaughter, when the animal is a bovine entire male, the dose rate is 0.10 to 0.15 mg/kg liveweight/day and the treatment continues for 25 days prior to slaughter, and, when the animal is a bovine castrated male, the dose rate is 0.06 to 0.10 mg/kg bodyweight/day and the treatment continues for 25 days prior to slaughter;

and wherein, when the animal is a female sheep, the dose rate is 0.06 to less than 0.10 mg/kg liveweight/day and the treatment continues for 25 days prior to slaughter, when the animal is an entire male sheep, the dose rate is 0.10 to 0.15 mg/kg liveweight/day and the treatment continues for 25 days prior to slaughter; and, when the animal is a castrated male sheep, the dose rate is 0.06 to 0.10 mg/kg liveweight/day and the treatment continues for 25 days prior to slaughter.

Preferably, when the animal is a castrated male pig, the treatment is continued for at least about 20 days prior to slaughter.

In a particularly preferred embodiment, the treatment is administered in a slow release form, for example in the form of implantable pellets or injectable pellets.

The methods of treatment described above may be provided utilising a veterinary composition including an aqueous buffer solution of 3.3 to 6.6 mg/ml of a source of growth hormone selected from exogenous, homologous growth hormone, analogues, derivatives or salts thereof.

The present invention will now be more fully described with reference to the accompanying examples.

The abbreviations used herein are defined as follows:

| | |
|---|---|
| GH | Growth hormone |
| ADG | Average daily liveweight gain (gm/day) |
| FCR | Feed conversion ratio (kg feed/kg liveweight gain) |
| ADFC | Average daily feed consumption (kg/day) |
| P2 Backfat | Depth of backfat at the P2 position (mm) |
| Lean tissue % | Percentage of evisoerated carcass with head off remaining after fat and bones have been removed |
| Dressing (%) | Ratio of hot carcass weight to final liveweight prior to slaughter. |

In each experiment, porcine GH, bovine GH and ovine GH prepared from fresh pituitaries by the method of Ellis (Endocrinology (1961) 69 554) was dissolved in buffer containing 0.05 M $Na_2CO_3$ and 0.05 M $NaHCO_3$, pH 10 to 11, and administered daily by intramuscular injection. Control animals received intramuscular injections of the same buffer containing 1% lactose. Unless otherwise stated, the pigs were fed ad libitum on a standard high energy diet supplying 14.3 MJ/kg. Sheep and cattle were fed ad libitum a commerical concentrate diet. Treatment commenced when the animals were approximately the weight stated in each case, and continued until they attained their slaughter weight. The animals were deprived of food and water for 24 hours before slaughter to enable an empty liveweight to be measured.

## Example 1 - Dose of Growth Hormone

In order to establish the optimum dose of GH, male pigs initially weighing 35 to 60 kg were treated with GH at dose of 0.0165, 0.033, 0.066, 0.099 and 0.132 mg/kg/day.

As the lowest doses tested, 0.0165 and 0.033 mg/kg/day there is no increase in ADG. Their doses offer no improvement in carcass quality and feed requirements are unchanged.

At a dose of 0.066 mg/kg/day male pigs showed a marked improvement in growth rate and FCR and reduction in backfat (see Tables 1 and 3). At a higher dose of 0.132 mg/kg/day growth rate was not further improved compared with a dose of 0.066 mg/kg/day whereas there was further improvement in FCR and a reduction in backfat at the higher dose (see Tables 6 and 8).

At a dose of 0.066 mg/kg/day female pigs also showed a marked improvement in growth rate and FCR and reduction in backfat (see Tables 2 and 4). At a higher dose of 0.132 mg/kg/day growth rate was not further improved compared with a dose of 0.066 mg/kg/day and there was only a marginal improvement in FCR and a minor reduction in backfat at the higher dose (see Tables 7 and 9).

Thus, there is only a narrow dose range within which improvement in carcass quality occurs simultaneously with improved growth rate and FCR.

## Example 2 - Time of Commencing Treatment

If treatment is given over the entire range of 35 to 90 kg, a still further improvement in FCR is observed compared to treatment over the 60 to 90 kg range. However, there is a greater relative improvement in FCR when GH is administered over the liveweight range of 60 to 90 kg than over the range 35 to 50 kg.

Furthermore, the improvement in carcass parameters (increase in lean percentage and decrease in fat percentage) is the same for treatment given only over the 60 to 90 kg range as it is if given over the whole 35 to 90 kg range (Dose of GH 0.066 mg/kg/day).

Results are shown in Tables 1 to 4.

## Example 3 - Effect of Sex and Diet

Results are provided in Tables 6 to 9.

In male pigs, treatment with GH at 0.066 or 0.132 mg/kg/day results in a greater improvement in FCR when the animals are fed on a relatively high energy diet, supplying 14.3 MJ/kg, compared to the relatively low energy diet supplying 13.0 MJ/kg. The compositions of the two diets are shown in Table 5. Each diet provided adequate protein for the animals.

Similarly, in female pigs there is greater improvement in FCR when fed the high energy diet, except where treatment is initiated at 70 kg, where the greater improvement occurs on the low energy diet.

For pigs of both sexes, the decrease in backfat thickness was greater on the low energy diet.

These treatments were repeated utilising synthetic recombinant porcine growth hormones (methionyl AA1-190 and AA4-190). Results showed a similar improvement in feed conversion efficiency and growth rate.

Example 4 - Effect of initiation time on growth rate, feed efficiency and carcass quality in castrate males

Table 10 illustrates that in castrate males, feed conversion efficiency and growth rate are best when animals are treated over the 50 to 90 kg treatment range.

Fat reduction as indicated by P2 backfat is effectively achieved at a dose rate of 0.066 and is only required over the last 10 kg of growth or over approximately the last 10 days prior to slaughter.

Example 5 - Effect of dose rate and treatment time on carcass quality

Tables 11 and 12 illustrate the anabolic effect of treatment with growth hormone on carcass quality whereby the weight of dissectable lean tissue is observed.

The results suggest treatment of both male and female pigs at the higher dose rate of 0.132 mg will provide the best anabolic effect.

Tables 13 and 14 illustrate the effectivenss of the fat reducing effect of growth hormone at higher treatment initiation weights.

The results suggest the higher dose rate of 0.132 mg/kg will cause the greatest reduction in carcass fat.

Example 6 - Effect of treatment time on growth rate, feed efficiency and carcass quality in cattle

Tables 15 to 18 illustrate that in cattle a treatment period of 50 days is to be preferred to obtain the best improvement in growth rate and feed conversion efficiency. However a greater relative improvement is achieved over the 25 day period.

Example 7 - Effect of dose rate and initiation time on sheep

Tables 19 to 24 illustrate that in sheep a treatment period of 50 days is to be preferred to maximise carcass quality and growth rate. A dose rate of 0.132 mg/kg provides the best carcass quality and growth rate improvement but the best relative improvement for female and male castrate sheep is achieved at the lower dose rate. However, the greater relative improvement is achieved over the 25 day period.

Example 8

Table 25 illustrates the variation in the amount of wool obtained from sheep treated with GH over 25-50 days. It may be postulated that the improvement in lean tissue content may be in an inverse relation to the reduction in wool yield. This may constitute a redirection of dietary nutrients.

## TABLE 1

Effect of daily GH treatment on male pigs initially weighing 35 kg.

|  | Control | Treatment | difference |
| --- | --- | --- | --- |
| Number of animals | 6 | 6 | |
| Dose of GH (mg/kg/day) | 0 | 0.066 | |
| ADG (gm/day) | 971 | 1048 | + 7.9% |
| FCR (kg feed/kg weight gain) | 2.40 | 2.16 | -10.0% |
| ADFC (kg/day) | 2.5 | 2.3 | - 8.7% |
| P2 Backfat (mm) | 22.0 | 18.3 | - 3.7 |
| Fat % | 31.6 | 25.9 | - 5.7 |
| Lean % | 65.7 | 71.4 | + 5.7 |
| Loin eye muscle length (mm) | 79.5 | 86.5 | + 7.0 |

## TABLE 2

Effect of daily GH treatment on female pigs initially weighing 35 kg.

| | Control | Treatment | difference |
|---|---|---|---|
| Number of animals | 6 | 6 | |
| Dose of GH (mg/kg/day) | 0 | 0.066 | |
| ADG (gm/day) | 834 | 939 | +12.6% |
| FCR (kg feed/kg weight gain) | 2.77 | 2.29 | -17.3% |
| ADFC (kg/day) | 2.31 | 2.15 | - 6.9% |
| P2 Backfat (mm) | 24.0 | 16.4 | - 7.6 |
| Fat % | 35.2 | 25.2 | -10.0 |
| Lean % | 62.9 | 73.1 | +10.2 |
| Loin eye muscle length (mm) | 78.1 | 92.7 | +14.6 |

## TABLE 3

Effect of daily GH treatment on male pigs initially weighing 60 kg.

|  | Control | Treated(1) | difference |
|---|---|---|---|
| Number of animals | 40 | 16 |  |
| Dose of GH (mg/kg/day) | 0 | 0.066 |  |
| ADG (gm/day) | 970 | 1030 | + 6.2% |
| FCR (kg feed/kg weight gain) | 2.97 | 2.62 | -11.8% |
| ADFC (kg/day) | 2.88 | 2.70 | - 6.3% |
| P2 Backfat (mm) | 21.1 | 15.5 | - 5.6 |
| Fat % | 29.5 | 21.6 | - 7.9 |
| Lean % | 67.8 | 75.7 | + 7.9 |

## TABLE 4

Effect of daily GH treatment on female pigs initially weighing 60 kg.

|  | Control | Treatment | difference |
|---|---|---|---|
| Number of animals | 8 | 8 | |
| Dose of GH (mg/kg/day) | 0 | 0.066 | |
| ADG (gm/day) | 855 | 1077 | +26.0% |
| FCR (kg feed/kg weight gain) | 2.98 | 2.27 | -23.8% |
| ADFC (kg/day) | 2.55 | 2.44 | - 4.3% |
| P2 Backfat (mm) | 24.1 | 16.5 | - 7.6 |
| Fat % | 34.9 | 23.2 | -11.7 |
| Lean % | 62.4 | 74.1 | +11.7 |

## TABLE 5

| | 14.3 MJ/kg HIGH ENERGY DIET % | | | 13.00 MJ/kg LOWER ENERGY DIET % | |
|---|---|---|---|---|---|
| 1. | WHEAT | 64.09 | 1. | BARLEY | 35.42 |
| 2. | RICE POLLARD | 15.00 | 2. | WHEAT | 23.33 |
| 3. | SOYA (48%) | 7.33 | 3. | MILLMIX | 20.00 |
| 4. | MILLMIX | 6.00 | 4. | RICE POLLARD | 15.00 |
| 5. | MEAT MEAL | 3.00 | 5. | BLOODMEAL | 2.33 |
| 6. | BLOODMEAL | 2.33 | 6. | LIME | 2.00 |
| 7. | LIME | 1.67 | 7. | SOYA (48%) | 1.33 |
| 8. | SALT | 0.24 | 8. | L-LYSINE | 0.26 |
| 9. | L-LYSINE | 0.22 | 9. | SALT | 0.20 |
| 10. | GROWER PREMIX | 0.12 | 10. | GROWER PREMIX | 0.12 |
| 11. | DL-METHIONINE | 0.00 | 11. | DL-METHIONINE | 0.01 |

12

## TABLE 6

Effect of daily GH treatment on male pigs initially weighing 50 kg, fed ad libitum two different diets and slaughtered at 90 kg liveweight.

| Diet (MJ/KG) | Control | Treated(1) | Treated(2) |
|---|---|---|---|
| 13.0 | | | |
| Number of animals | 7 | 8 | 5 |
| Dose of GH (mg/kg/day) | 0 | 0.066 | 0.132 |
| Average Daily gain (gm/day) | 958 | 1073 | 1088 |
| Feed conversion ratio | 3.26 | 3.04 | 2.79 |
| P2 Backfat | 15.2 | 14.1 | 12.7 |
| 14.3 | | | |
| Number of animals | 6 | 8 | 7 |
| Dose of GH (mg/kg/day) | 0 | 0.066 | 0.132 |
| Average daily gain (gm/day) | 1067 | 1167 | 1187 |
| Feed conversion ratio | 3.01 | 2.57 | 2.48 |
| P2 Backfat (mm) | 17.4 | 16.5 | 14.8 |

13

## TABLE 7

Effect of daily GH treatment on female pigs initially weighing 50 kg, fed ad libitum two different diets and slaughtered at 90 kg liveweight.

| | Diet (MJ/KG) | Control | Treated(1) | Treated(2) |
|---|---|---|---|---|
| | 13.0 | | | |
| Number of animals | | 4 | 7 | 8 |
| Dose of GH (mg/kg/day) | | 0 | 0.066 | 0.132 |
| Average daily gain (gm/day) | | 902 | 962 | 966 |
| Feed conversion ratio | | 3.64 | 3.24 | 3.33 |
| P2 Backfat (mm) | | 19.6 | 13.6 | 12.5 |
| | 14.3 | | | |
| Number of animals | | 7 | 8 | 6 |
| Dose of GH (mg/kg/day) | | 0 | 0.066 | 0.132 |
| Average daily gain (gm/day) | | 923 | 1028 | 1007 |
| Feed conversion ratio | | 3.46 | 3.00 | 2.79 |
| P2 Backfat (mm) | | 20.2 | 16.3 | 14.1 |

## TABLE 8

Effect of daily GH treatment on male pigs initially weighing 70 kg, fed ad libitum two different diets and slaughtered at 90 kg liveweight.

| Diet (MJ/KG) | Control | Treated(1) | Treated(2) |
|---|---|---|---|
| **13.0** | | | |
| Number of animals | 7 | 7 | 8 |
| Dose of GH (mg/kg/day) | 0 | 0.066 | 0.132 |
| Average daily gain (gm/day) | 1011 | 1087 | 1072 |
| Feed conversion ratio | 3.55 | 2.92 | 2.89 |
| P2 Backfat (mm) | 17.4 | 14.6 | 12.1 |
| **14.3** | | | |
| Number of animals | 8 | 6 | 8 |
| Dose of GH (mg/kg/day) | 0 | 0.066 | 0.132 |
| Average daily gain (gm/day) | 1070 | 1206 | 1146 |
| Feed conversion ratio | 3.23 | 2.67 | 2.56 |
| P2 Backfat (mm) | 18.4 | 16.7 | 5.2 |

## TABLE 9

Effect of daily GH treatment on female pigs initially weighing 70 kg, fed ad libitum two different diets.

| Diet (MJ/KG) | Control | Treated(1) | Treated(2) |
|---|---|---|---|
| **13.0** | | | |
| Number of animals | 7 | 6 | 7 |
| Dose of GH (mg/kg/day) | 0 | 0.066 | 0.132 |
| Average daily gain (gm/day) | 796 | 909 | 908 |
| Feed conversion ratio | 4.23 | 3.17 | 3.08 |
| P2 Backfat (mm) | 19.3 | 15.6 | 14.2 |
| **14.3** | | | |
| Number of animals | 6 | 7 | 7 |
| Dose of GH (mg/kg/day) | 0 | 0.066 | 0.132 |
| Average daily gain (gm/day) | 902 | 1027 | 927 |
| Feed conversion ratio | 3.62 | 3.01 | 2.82 |
| P2 Backfat (mm) | 19.7 | 17.4 | 15.1 |

## TABLE 10

Effect of daily GH treatment on castrate pigs, initially weighing 50 kg and treated from 50, 70 and 80 kg to slaughter fed ad libitum a 14.3 MJ/kg diet.

|  | Control | Treated(1) | Treated(2) | Treated(3) |
|---|---|---|---|---|
| Number of animals | 8 | 8 | 8 | 8 |
| Weight range of GH administration * (kg) | Nil | 80-90 | 70-90 | 50-90 |
| Average daily gain (gm/day) | 905 | 923 | 1060 | 1131 |
| Feed conversion ratio | 3.60 | 3.23 | 2.93 | 2.66 |
| P2 Backfat (mm) | 24.1 | 19.4 | 18.6 | 19.2 |

\*    Dose rate 0.066 mg/kg/day

## TABLE 11

Effect of daily GH treatment on male pigs initially weighing 50 kg.

|                      | Control | Treated(1) | Treated(2) |
|----------------------|---------|------------|------------|
| Number of animals    | 7       | 8          | 8          |
| Dose of GH (mg/kg/day) | 0     | 0.066      | 0.132      |
| Shoulder - fat (kg)  | 1.98    | 1.69       | 1.82       |
| - bone (kg)          | 1.65    | 1.53       | 1.77       |
| - lean (kg)          | 5.13    | 5.13       | 5.28       |
| Loin - fat (kg)      | 1.83    | 1.77       | 1.59       |
| - bone (kg)          | 1.04    | 0.96       | 1.18       |
| - lean (kg)          | 3.76    | 3.88       | 4.06       |
| Leg - fat (kg)       | 2.09    | 1.92       | 1.77       |
| - bone (kg)          | 1.80    | 1.82       | 1.97       |
| - lean (kg)          | 5.43    | 5.85       | 5.63       |

Weights of tissues relate to half the carcass

Pigs fed ad libitum 14.3 MJ/kg diet

## TABLE 12

Effect of daily GH treatment on female pigs initially weighing 50 kg.

|  | | Control | Treated(1) | Treated(2) |
|---|---|---|---|---|
| Number of animals | | 7 | 8 | 5 |
| Dose of GH (mg/kg/day) | | 0 | 0.066 | 0.132 |
| Shoulder | - fat (kg) | 2.29 | 1.98 | 1.68 |
| | - bone (kg) | 1.54 | 1.51 | 1.63 |
| | - lean (kg) | 4.95 | 5.09 | 5.42 |
| Loin | - fat (kg) | 2.21 | 1.60 | 1.46 |
| | - bone (kg) | 0.98 | 0.97 | 1.05 |
| | - lean (kg) | 3.77 | 3.71 | 3.93 |
| Leg | - fat (kg) | 2.58 | 2.33 | 1.98 |
| | - bone (kg) | 1.62 | 1.76 | 1.82 |
| | - lean (kg) | 5.34 | 5.61 | 5.91 |

Weights of tissues relate to half the carcass

Pigs fed ad libitum 14.3 MJ/kg diet

## TABLE 13

Effect of daily treatment on male pigs initially weighing 70 kg.

|  |  | Control | Treated(1) | Treated(2) |
|---|---|---|---|---|
| Number of animals |  | 8 | 8 | 8 |
| Dose of GH (mg/kg/day) |  | 0 | 0.066 | 0.132 |
| Shoulder | - fat (%) | 23.5 | 19.7 | 19.9 |
|  | - bone (%) | 18.3 | 19.3 | 18.0 |
|  | - lean (%) | 58.2 | 61.0 | 62.1 |
| Loin | - fat (%) | 27.0 | 24.0 | 21.1 |
|  | - bone (%) | 15.6 | 15.5 | 15.6 |
|  | - lean (%) | 57.4 | 60.5 | 63.3 |
| Leg | - fat (%) | 23.0 | 19.8 | 17.7 |
|  | - bone (%) | 19.9 | 20.8 | 19.5 |
|  | - lean (%) | 57.1 | 59.4 | 62.8 |

Pigs fed ad libitum 13.0 MJ/kg diet

## TABLE 14

Effect of daily GH treatment on female pigs initially weighing 70 kg.

|  |  | Control | Treated(1) | Treated(2) |
|---|---|---|---|---|
| Number of animals |  | 4 | 5 | 5 |
| Dose of GH (mg/kg/day) |  | 0 | 0.066 | 0.132 |
| Shoulder | - fat (%) | 26.5 | 21.2 | 20.6 |
|  | - bone (%) | 16.7 | 17.8 | 18.3 |
|  | - lean (%) | 56.8 | 61.0 | 61.1 |
| Loin | - fat (%) | 31.0 | 26.1 | 23.2 |
|  | - bone (%) | 14.0 | 15.5 | 16.4 |
|  | - lean (%) | 55.0 | 58.4 | 60.4 |
| Leg | - fat (%) | 27.5 | 21.6 | 21.4 |
|  | - bone (%) | 17.3 | 17.8 | 18.3 |
|  | - lean (%) | 55.2 | 60.6 | 60.3 |

Pigs fed ad libitum 13.0 MJ/kg diet

## TABLE 15

Effect of daily GH treatment for 50 days on female cattle initially weighing approximately 200-220 kg fed _ad libitum_ and slaughtered after 50 days of treatment.

|  | Control | Treatment |
|---|---|---|
| No. of Animals | 7 | 7 |
| Av. Starting Weight (kg) | 224 | 206 |
| Av. Finishing Weight (kg) | 280 | 274 |
| Dose GH (mg/kg/day) | 0 | 0.066 |
| ADG (grams) | 1125 | 1354 |
| FCR (kg feed/kg weight gain) | 6.49 | 4.47 |
| Fat Scores (1) | 7.15    5.51 | 6.75   5.26 |
| Fat Scores (2) | 7.0 | 6.1 |
| Dressing % (hot carcass) | 57.8 | 57.5 |

(1)  The fat measured is taken at the 1/2 and 3.4 position (a point 1/2 and 3/4 of the breadth of the rib eye muscle away from the chine) between the 12th/13th rib.

(2)  Depth of fat at the 12th/13th rib using the CALM system of assessment

## TABLE 16

Effect of daily GH treatment for 50 days on female cattle initially weighing approximately 205 kg with placebo treatment for the first 25 days, followed by 25 days of treatment with GH. The cattle were fed _ad libitum_ and slaughtered at the end of the 50 day period.

| | Control | Treatment |
|---|---|---|
| No. of Animals | 7 | 7 |
| Av. Starting Weight at Day 0 (kg) | 224 | 205 |
| Av. Starting Weight at Day 25 (kg) | 248 | 227 |
| Av. Finishing Weight at Day 50 (kg) | 281 | 271 |
| Dose GH (mg/kg/day) | 0 | 0.066 |
| ADG for Days 25-50 (grams) | 1304 | 1739 |
| FCR for Days 25-50 (kg feed/kg weight gain) | 6.11 | 3.68 |
| Fat Scores (1) | 7.15    5.51 | 6.80    5.35 |
| Fat Score (2) | 7.0 | 6.5 |
| Dressing % (hot carcass) | 57.8 | 58.6 |

## TABLE 17

Effect of daily treatment for 50 days on castrated male cattle initially weighing approximately 210-220 kg fed ad libitum and slaughtered after 50 days of treatment.

|  | Control | Treatment |
|---|---|---|
| No. of Animals | 7 | 8 |
| Av. Starting Weight (kg) | 212 | 217 |
| Av. Finishing Weight (kg) | 277 | 293 |
| Dose of GH (mg/kg/day) | 0 | 0.066 |
| ADG (grams) | 1292 | 1520 |
| FCR (kg feed/kg weight gain) | 5.45 | 4.66 |
| Fat Scores (1) | 6.14   4.14 | 4.23   3.53 |
| Fat Score (2) | 6.9 | 5.9 |
| Dressing % (hot carcass) | 58.8 | 58.7 |

24

## TABLE 18

Effect of daily GH treatment for 25 days on castrated male cattle initially weighing approximately 225 kg with placebo treatment for the first 25 days followed by 25 days of treatment with GH. The cattle were fed _ad libitum_ and slaughtered at the end of the 50 day period.

|  | Control | | Treatment | |
| --- | --- | --- | --- | --- |
| No. of Animals | 7 | | 7 | |
| Av. Starting Weight at Day 0 (kg) | 212 | | 226 | |
| Av. Starting Weight at Day 25 (kg) | 241 | | 252 | |
| Av. Finishing Weight at Day 5 (kg) | 277 | | 302 | |
| Dose GH (mg/kg/day) | 0 | | 0.066 | |
| ADG for Days 25-50 (grams) | 1435 | | 2000 | |
| FCR for Days 25-50 (kg feed/kg weight gain) | 5.36 | | 3.58 | |
| Fat Scores (1) | 6.14 | 4.14 | 4.21 | 2.66 |
| Fat Score (2) | 6.9 | | 6.1 | |
| Dressing % (hot carcass) | 58.8 | | 59.6 | |

## TABLE 19

Effect of daily treatment on female sheep initially weighing approximately 20 kg fed _ad libitum_ after 50 days of treatment.

|  | Control | Treatment(1) | Treatment(2) |
|---|---|---|---|
| No. of Animals | 8 | 8 | 8 |
| Av. Starting Weight (kg) | 19.6 | 20.1 | 19.8 |
| Av. Finishing Weight (kg) | 29.1 | 31.1 | 31.8 |
| Dose of GH (mg/kg/day) | 0 | 0.066 | 0.132 |
| ADG (grams) | 190 | 220 | 240 |
| FCR (kg feed/kg live weight gain) | 4.65 | 3.74 | 3.55 |
| Fat Scores | C  GR | C  GR | C  GR |
|  | 2.6  17.1 | 2.3  15.2 | 3.0  16.0 |
| Dressing % (hot carcass) | 48.1 | 48.4 | 51.2 |

"C"   fat depth over eye muscle (30-40 mm from midline) measured between the 12th and 13th ribs

"GR"   depth of tissue 110 mm from midline measured between the 12th and 13th ribs

## TABLE 20

Effect of daily GH treatment for 25 days on female sheep initially weighing approximately 20 kg with placebo treatment for the first 25 days followed by 25 days of treatment with GH. The sheep were fed _ad libitum_ and slaughtered at the end of the 50 day period.

| | Control | Treatment(1) | Treatment(2) |
|---|---|---|---|
| No. of Animals | 8 | 8 | 8 |
| Av. Starting Weight at Day 0 (kg) | 19.6 | 19.5 | 20.8 |
| Av. Starting Weight at Day 25 (kg) | 23.9 | 24.0 | 25.3 |
| Av. Finishing Weight at Day 50 (kg) | 29.1 | 32.0 | 33.8 |
| Dose of GH (mg/kg/day) | 0 | 0.066 | 0.132 |
| ADG for Days 25-50 (grams) | 210 | 320 | 340 |
| FCR for Days 25-50 (kg feed/kg live weight gain) | 4.65 | 3.35 | 3.21 |

| | C | GR | C | GR | C | GR |
|---|---|---|---|---|---|---|
| Fat Scores | 2.6 | 17.1 | 1.8 | 15.9 | 2.5 | 16.0 |

| | Control | Treatment(1) | Treatment(2) |
|---|---|---|---|
| Dressing % (hot carcass) | 48.1 | 49.1 | 49.3 |

## TABLE 21

Effect of daily GH treatment on castrated male sheep initially weighing approximately 20 kg fed <u>ad libitum</u> and slaughtered after 50 days of treatment.

|  | Control | Treatment(1) | Treatment(2) |
|---|---|---|---|
| No. of Animals | 8 | 8 | 8 |
| Av. Starting Weight | 19.5 | 20.3 | 20.4 |
| Av. Finishing Weight | 31.0 | 33.3 | 32.4 |
| Dose GH (mg/kg/day) | 0 | 0.066 | 0.132 |
| ADG (grams) | 230 | 260 | 240 |
| FCR (kg feed/kg live weight gain) | 3.95 | 3.40 | 3.58 |

| Fat Scores | C | GR | C | GR | C | GR |
|---|---|---|---|---|---|---|
|  | 3.8 | 19.8 | 2.5 | 14.5 | 1.9 | 14.6 |

|  | Control | Treatment(1) | Treatment(2) |
|---|---|---|---|
| Dressing % (hot carcass) | 49.9 | 46.2 | 50.9 |

## TABLE 22

Effect of daily GH treatment for 25 days on castrated male
sheep initially weighing approximately 20 kg with placebo
treatment for the first 25 days followed by 25 days of
treatment with GH.  The sheep were fed _ad libitum_ and
slaughtered at the end of the 50 day period.

|  | Control | Treatment(1) | Treatment(2) |
|---|---|---|---|
| No. of Animals | 8 | 8 | 8 |
| Av. Starting Weight at Day 0 (kg) | 19.5 | 20.8 | 20.2 |
| Av. Starting Weight at Day 25 (kg) | 24.0 | 25.0 | 24.5 |
| Av. Finishing Weight at Day 50 (kg) | 31.0 | 33.3 | 33.3 |
| Dose GH (mg/kg/day) | 0 | 0.066 | 0.132 |
| ADG for Days 25-50 (grams) | 280 | 330 | 350 |
| FCR for Days 25-50 (kg feed/kg live weight gain) | 3.90 | 3.46 | 3.17 |
| Fat Scores | C  GR<br>3.8  19.8 | C  GR<br>2.5  15.9 | C  GR<br>2.8  17.6 |
| Dressing % (hot carcass) | 49.9 | 49.3 | 50.9 |

## TABLE 23

Effect of daily GH treatment on entire male sheep initially weighing approximately 15-20 kg fed _ad libitum_ and slaughtered after 50 days of treatment.

|  | Control | Treatment(1) | Treatment(2) |
|---|---|---|---|
| No. of Animals | 6 | 6 | 6 |
| Av. Starting Weight (kg) | 15.2 | 20.0 | 17.5 |
| Av. Finishing Weight (kg) | 31.0 | 35.5 | 34.7 |
| Dose of GH (mg/kg/day) | 0 | 0.066 | 0.132 |
| ADG (grams) | 315 | 310 | 344 |
| FCR (kg feed/kg live weight gain) | 3.16 | 3.46 | 3.15 |
| Fat Scores | C    GR<br>3.3    14.9 | C    GR<br>2.4    14.4 | C    GR<br>2.1    12.6 |
| Dressing % (hot carcass) | 47.9 | 48.7 | 48.1 |

## TABLE 24

Effect of daily GH treatment for 25 days on entire male sheep initially weighing approximately 15-18 kg with placebo treatment for the first 25 days followed by 25 days of treatment with GH.  The sheep were fed ad libitum and slaughtered at the end of the 50 day period.

|  | Control | Treatment(1) | Treatment(2) |
|---|---|---|---|
| No. of Animals | 6 | 6 | 6 |
| Av. Starting Weight at Day 0 (kg) | 15.2 | 17.6 | 16.0 |
| Av. Starting Weight at Day 25 (kg) | 22.5 | 24.6 | 23.3 |
| Av. Finishing Weight at Day 50 (kg) | 31.0 | 33.7 | 32.7 |
| Dose GH (mg/kg/day) | 0 | 0.066 | 0.132 |
| ADG for Days 25-30 (grams) | 343 | 363 | 376 |
| FCR for Days 25-50 (kg feed/kg weight gain) | 3.70 | 3.73 | 3.47 |

| | Control | | Treatment(1) | | Treatment(2) | |
|---|---|---|---|---|---|---|
| Fat Scores | C | GR | C | GR | C | GR |
| | 3.3 | 14.9 | 3.1 | 12.9 | 2.1 | 12.7 |

| | Control | Treatment(1) | Treatment(2) |
|---|---|---|---|
| Dressing % (hot carcass) | 47.9 | 48.1 | 48.7 |

31

EP 0 217 572 B1

## TABLE 25

Amount of wool expressed as grams/kg live weight obtained from sheep treated with GH for 25-50 days. 8 sheep per group.

|  | Castrated Males | Females | Entire Males |
|---|---|---|---|
| Control | 39 | 41 | 41 |
| Treated (1) for last 50 days | 35 | 41 | 33 |
| Treated (2) for last 50 days | 36 | 38 | 34 |
| Treated (1) for last 25 days | 35 | 34 | 31 |
| Treated (2) for last 25 days | 35 | 34 | 31 |

(1)  0.066 mg GH/kg liveweight/day
(2)  0.132 mg GH/kg liveweight/day

**Claims**

1. A method for the treatment of an animal to improve carcass quality and food conversion efficiency, which method is characterised in that an exogenous, homologous growth hormone selected from natural or synthetic growth hormone, analogues, derivatives, or fragments thereof is administered to an animal to be treated selected from entire male, female and castrated male pigs, entire male, female and castrated male sheep, and entire male, female and castrated male bovine animals at a preselected liveweight and at a preselected constant dose rate depending on the species and sex of the animal, and in the absence of zearalin;

   wherein, when the animal is a pig, the administration is initiated at a liveweight of 35 to 100 kg, and, when the pig is female or a castrated male, the dose rate is 0.06 to 0.10 mg/kg liveweight/day, and, when the pig is an entire male, the dose rate is 0.1 to 0.15 mg/kg liveweight/day;

   wherein, when the animal is a bovine animal, administration is initiated at a bodyweight of 200 to 500 kg, and, when the bovine animal is a female or a castrated male, the dose rate is 0.06 to 0.10 mg/kg liveweight/day, and, when the bovine animal is an entire male, the dose rate is 0.10 to 0.15 mg/kg liveweight/day,

   and wherein, when the animal is a sheep, administration is initiated at a bodyweight of 15 to 50 kg, and, when the sheep is female or a castrated male, the dose rate is 0.06 to less than 0.10 mg/kg liveweight/day, and, when the sheep is an entire male, the dose rate is 0.10 to 0.15 mg/kg liveweight/day.

2.  A method according to claim 1, wherein, when the animal is a pig, the administration is initiated at a liveweight of 50 to 100 kg.

3.  A method according to claim 1, wherein, when the animal is a bovine animal, the administration is initiated at a liveweight of 250 to 300 kg.

4.  A method according to claim 1, wherein, when the animal is a sheep, the administration is initiated at a liveweight of 25 to 35 kg.

5.  A method of decreasing fat content of an animal carcass, which method is characterised in that an exogenous, homologous growth hormone selected from natural or synthetic growth hormone, analogues, derivatives or fragments thereof is administered to an animal to be treated at a generally constant dose rate and in the absence of zearalin;
    wherein, when the animal is a female pig, the dose rate is 0.06 to 0.1 mg/kg liveweight/day and the treatment continues for approximately 20 days prior to slaughter, when the animal is an entire male pig, the dose rate is 0.1 to 0.15 mg/kg liveweight/day and the treatment continues for approximately 20 days prior to slaughter, and, when the animal is a castrated male pig, the dose rate is 0.06 to 0.1 mg/kg liveweight/day and the treatment continues for approximately 10 days to at least about 20 days prior to slaughter;
    wherein, when the animal is a bovine female, the dose rate is 0.06 to 0.10 mg/kg liveweight/day and the treatment continues for 25 days prior to slaughter, when the animal is a bovine entire male, the dose rate is 0.10 to 0.15 mg/kg liveweight/day and the treatment continues for 25 days prior to slaughter, and, when the animal is a bovine castrated male, the dose rate is 0.06 to 0.10 mg/kg bodyweight/day and the treatment continues for 25 days prior to slaughter;
    and wherein, when the animal is a female sheep, the dose rate is 0.06 to less than 0.10 mg/kg liveweight/day and the treatment continues for 25 days prior to slaughter, when the animal is an entire male sheep, the dose rate is 0.10 to 0.15 mg/kg liveweight/day and the treatment continues for 25 days prior to slaughter; and, when the animal is a castrated male sheep, the dose rate is 0.06 to 0.10 mg/kg liveweight/day and the treatment continues for 25 days prior to slaughter.

6.  A method according to claim 5, wherein, when the animal is a castrated male pig, the treatment is continued for at least about 20 days prior to slaughter.

**Patentansprüche**

1.  Verfahren zur Behandlung eines Tiers zur Verbesserung der Qualität des Tierkörpers und der Wirksamkeit der Futterverwertung, dadurch gekennzeichnet, daß ein exogenes, homologes Wachstumshormon, ausgewählt aus natürlichem oder synthetischem Wachstumshormon, Analogen, Derivaten oder Fragmenten hiervon, einem zu behandelndem Tier mit einer im allgemeinen konstanten Dosisrate und in Abwesenheit von Zearalin verabreicht wird;
    worin, wenn das Tier ein Schwein ist, mit der Verabreichung bei einem Lebendgewicht von 35 bis 100 kg begonnen wird und, wenn das Schwein ein weibliches oder ein kastriertes männliches Tier ist, die Dosisrate 0,06 bis 0,10 mg/kg Lebendgewicht /Tag beträgt und, wenn das Schwein ein nichtkastriertes männliches Tier ist, die Dosisrate 0,1 bis 0,15 mg/kg Lebendgewicht/Tag beträgt;
    worin, wenn das Tier ein Rind ist, mit der Verabreichung bei einem Körpergewicht von 200 bis 500 kg begonnen wird und, wenn das Rind ein weibliches oder ein kastriertes männliches Tier ist, die Dosisrate 0,06 bis 0,10 mg/kg Lebendgewicht/Tag beträgt und, wenn das Rind ein nichtkastriertes männliches Tier ist, die Dosisrate 0,10 bis 0,15 mg/kg Lebendgewicht/Tag beträgt; und
    worin, wenn das Tier ein Schaf ist, mit der Behandlung bei einem Körpergewicht von 15 bis 50 kg begonnen wird, und, wenn das Schaf ein weibliches oder ein kastriertes männliches Tier ist, die Dosisrate 0,06 bis weniger als 0,10 mg/kg Lebendgewicht/Tag berägt, und, wenn das Schaf ein unkastriertes männliches Tier ist, die Dosisrate 0,10 bis 0,15 mg/kg Lebendgewicht /Tag beträgt.

2.  Verfahren nach Anspruch 1, worin, wenn das Tier ein Schwein ist, mit der Verabreichung bei einem Lebendgewicht von 50 bis 100 kg begonnen wird.

3. Verfahren nach Anspruch 1, worin, wenn das Tier ein Rind ist, mit der Verabreichung bei einem Lebendgewicht von 250 bis 300 kg begonnen wird.

4. Verfahren nach Anspruch 1, worin, wenn das Tier ein Schaf ist, mit der Verabreichung bei einem Lebendgewicht von 25 bis 35 kg begonnen wird.

5. Verfahren zur Herabsetzung des Fettgehalts eines Tierkörpers, dadurch gekennzeichnet, daß ein exogenes, homologes Wachstumshormon, ausgewählt aus natürlichem oder synthetischem Wachstumshormon, Analogen, Derivaten oder Fragmenten hiervon einem zu behandelnden Tier mit einer im allgemeinen konstanten Dosisrate und in Abwesenheit von Zearalin verabreicht wird;
worin, wenn das Tier ein weibliches Schwein ist, die Dosisrate 0,06 bis 0,1 mg/kg Lebendgewicht/Tag beträgt und die Behandlung über etwa 20 Tage vor dem Schlachten durchgeführt wird, wenn das Tier ein nichtkastriertes männliches Schwein ist, die Dosisrate 0,1 bis 0,15 mg/kg Lebendgewicht/ Tag beträgt und die Behandlung über etwa 20 Tage vor dem Schlachten durchgeführt wird, und, wenn das Tier ein kastriertes männliches Schwein ist, die Dosisrate 0,06 bis 0,1 mg/kg Lebendgewicht /Tag beträgt und die Behandlung über etwa 10 Tage bis mindestens etwa 20 Tage vor dem Schlachten durchgeführt wird;
worin, wenn das Tier ein weibliches Rind ist, die Dosisrate 0,06 bis 0,10 mg/kg Lebendgewicht/Tag beträgt und die Behandlung über 25 Tage vor dem Schlachten durchgeführt wird, wenn das Tier ein nichtkastriertes männliches Rind ist, die Dosisrate 0,10 bis 0,15 mg/kg Lebendgewicht/Tag beträgt und die Behandlung über 25 Tage vor dem Schlachten durchgeführt wird und, wenn das Tier ein kastriertes männliches Rind ist, die Dosisrate 0,06 bis 0,10 mg /kg Körpergewicht/Tag beträgt und die Behandlung über 25 Tage vor dem Schlachten durchgeführt wird; und,
worin, wenn das Tier ein weibliches Schaf ist, die Dosisrate 0,06 bis weniger als 0,10 mg/kg Lebendgewicht/Tag beträgt und die Behandlung über 25 Tage vor dem Schlachten durchgeführt wird, wenn das Tier ein nichtkastriertes männliches Schaf ist, die Dosisrate 0,10 bis 0,15 mg/kg Lebendgewicht/Tag beträgt und die Behandlung über 25 Tage vor dem Schlachten durchgeführt wird, und, wenn das Tier ein kastriertes männliches Schaf ist, die Dosisrate 0,06 bis 0,10 mg/kg Lebendgewicht/Tag beträgt und die Behandlung über 25 Tage vor dem Schlachten durchgeführt wird.

6. Verfahren nach Anspruch 5, worin, wenn das Tier ein kastriertes männliches Schwein ist, die Behandlung über mindestens etwa 20 Tage vor dem Schlachten durchgeführt wird.

**Revendications**

1. Procédé pour le traitement d'un animal afin d'améliorer la qualité de carcasse et le rendement de conversion alimentaire, ce procédé étant caractérisé en ce qu'une hormone de croissance homologue exogène choisie parmi une hormone de croissance naturelle ou synthétique, des analogues de cette hormone, des hormones dérivées de celle-ci ou des fragments de celle-ci est administrée à un animal qui doit être traité et qui est choisi parmi des porcs entiers mâles, femelles et mâles castrés, parmi des moutons entiers mâles, femelles et mâles castrés, parmi des animaux bovins entiers mâles, femelles et mâles castrés, cet animal étant choisi pour un poids sur pieds ou poids vif présélectionné et l'hormone de croissance étant administrée selon une posologie constante présélectionné en fonction de l'espèce et du sexe de l'animal et de l'absence de zéaraline ;
dans lequel, lorsque l'animal est un porc, l'administration est démarrée à un poids sur pied de 35 à 100 kg, lorsque le porc est une femelle ou un mâle castré, la posologie est de 0,06 à 0,10 mg/kg de poids sur pied/jour et lorsque le porc est un mâle entier, la posologie est de 0,1 à 0,15 mg/kg de poids sur pied/jour ;
dans lequel, lorsque l'animal est un bovin, l'administration est démarrée pour un poids sur pied de 200 à 500 kg, lorsque l'animal bovin est une femelle ou un mâle castré, la posologie est de 0,06 à 0,10 mg/kg de poids sur pied/jour et lorsque l'animal bovin est un mâle entier, la posologie est de 0,10 à 0,15 mg/kg de poids sur pied/jour ;
et dans lequel, lorsque l'animal est un mouton, l'administration est démarrée pour un poids sur pied de 15 à 50 kg, lorsque le mouton est une femelle ou un mâle castré, la posologie est de 0,06 jusqu'à moins de 0,10 mg/kg de poids sur pied/jour et lorsque le mouton est un mâle entier, la posologie est de 0,10 à 0,15 mg/kg de poids sur pied/jour.

2. Procédé selon la revendication 1, dans lequel, lorsque l'animal est un porc, l'administration est commencée à un poids sur pied de 50 à 100 kg.

3. Procédé selon la revendication 1, dans lequel, lorsque l'animal est un animal bovin, l'administration est commencée à un poids sur pied de 250 à 300 kg.

4. Procédé selon la revendication 1, dans lequel, lorsque l'animal est un mouton, l'administration est commencée à un poids sur pied de 25 à 35 kg.

5. Procédé de diminution de la teneur en graisse d'une carcasse d'animal, ce procédé étant caractérisé en ce qu'une hormone de croissance homologue exogène choisie parmi une hormone de croissance naturelle ou synthétique, des analogues de cette hormone, des hormones dérivées de celle-ci ou des fragments de celle-ci est administrée à un animal qui doit être traité selon une posologie généralement constante et en l'absence de zéaraline ;

dans lequel, lorsque l'animal est un porc femelle, la posologie est de 0,06 à 0,1 mg/kg de poids sur pied/jour et le traitement se poursuit pendant approximativement 20 jours avant l'abattage, lorsque l'animal est un porc mâle entier, la posologie est de 0,1 à 0,15 mg/kg de poids sur pieds/jour et le traitement se poursuit pendant approximativement 20 jours avant l'abattage et lorsque l'animal est un porc mâle castré, la posologie est de 0,06 à 0,1 mg/kg de poids sur pied/jour et le traitement se poursuit pendant approximativement 10 jours jusqu'à au moins environ 20 jours avant l'abattage ;

dans lequel, lorsque l'animal est une femelle bovine, la posologie est de 0,06 à 0,10 mg/kg de poids sur pied/jour et le traitement se poursuit pendant 25 jours avant l'abattage, lorsque l'animal est un mâle entier bovin, la posologie est de 0,10 à 0,15 mg/kg de poids sur pied/jour et le traitement se poursuit pendant 25 jours avant l'abattage et lorsque l'animal est un mâle castré bovin, la posologie est de 0,06 à 0,10 mg/kg de poids sur pied/jour et le traitement se poursuit pendant 25 jours avant l'abattage ;

et dans lequel, lorsque l'animal est un mouton femelle, la posologie est de 0,06 jusqu'à moins de 0,10 mg/kg de poids sur pied/jour et le traitement se poursuit pendant 25 jours avant l'abattage, lorsque l'animal est un mouton mâle entier, la posologie est de 0,10 à 0,15 mg/kg de poids sur pied/jour et le traitement se poursuit pendant 25 jours avant l'abattage ; et lorsque l'animal est un mouton mâle castré, la posologie est de 0,06 à 0,10 mg/kg de poids sur pied/jour et le traitement se poursuit pendant 25 jours avant l'abattage.

6. Procédé selon la revendication 5, dans lequel, lorsque l'animal est un porc mâle castré, le traitement est poursuivi pendant au moins environ 20 jours avant l'abattage.